# EUROPEAN PATENT APPLICATION

(11) **EP 3 698 836 A1**
(43) Date of publication of application: **26.08.2020**
(21) Application number: 19382135.2
(22) Date of filing: 25.02.2019
(51) Int. Cl.: A61M 16/04, A61M 25/00, A61M 16/20, A61B 1/267

(54) **LARYNGEAL MASK**

(71) Applicant: Visual Oxy, S.L., Málaga (ES)
(72) Inventor: MONTERO GONZÁLEZ, Emilio, 29002 Málaga (ES)
(74) Representative: Elzaburu S.L.P.

(57) **Abstract**

Laryngeal mask (1) comprising a dome (5) and a plurality of tubes for the airways connected to said mask, wherein the axes of the tubes are parallel, have an axial eccentricity, and at least one of them has a section with an irregular, asymmetric and non-circular geometric shape.

Laryngeal mask (1) comprising a dome (5) and a connecting channel configured to produce the artificial ventilation of a patient, wherein the laryngeal mask (1) further comprises an inlet (9) of a gastric aspiration tube, said inlet (9) being located at a distal end of said dome (5), on the side wall of this dome (5), wherein said inlet (9) is configured to absorb any gastric secretions from the patient's oesophagus (3), wherein the laryngeal mask (1) comprises at least a non-return valve (4) located at the distal end of the dome (5), wherein the valve (4) comprises at least two flexible membranes configured to allow fluid to flow in a single direction from the outside to the inside of the dome (5), wherein the pipes of the connecting channel connecting the dome (5) with the artificial respirator outside have in their section a geometric configuration classified as an irregular, asymmetric and non-circular polygon.

## Description

### Object of the invention

The present invention concerns with a laryngeal mask incorporating a series of improvements over the prior art, including a disposition of irregular and asymmetrical eccentric parallel tubes that save valuable space within the larynx, as well as a non-return valve designed to prevent effluent gastric juices from the oesophagus from entering the trachea during an operation involving the pumping of oxygen into the lungs of the patient by means of a laryngeal mask, among others.

The laryngeal mask object of this invention has special application in the field of industry dedicated to the design, manufacture and marketing of surgical instruments, especially instruments intended to pump air into the lungs of a patient.

### Background of the invention and technical problem to be solved

In the hospital and out-of-hospital world, the provision of correct patient ventilation is the first point to be taken into account as an initial step for the treatment of any patient, regardless of the pathology they have. When a patient is unconscious due to anaesthesia or unconscious due to an accident or illness, ventilation will be the most important point to keep the patient alive.

In the state of the art there are currently only two ways to ventilate an unconscious patient with anaesthesia or unconscious by accident or disease: one is to insert an endotracheal tube through the trachea and ventilate the patient, and the other method is to use a laryngeal mask in the patient's larynx to ventilate the patient.

At present, the use of one or another method is more or less balanced in percentage, 50/50, varying a little depending on the cause, the pathology, the duration of the surgery, the severity of the patient, the ability of the doctor, if its use is in an emergency or in an operating room, or a one-day ambulatory surgery, etc.

Currently, one method that exists to practice said ventilation of the patient is to integrate a gastric aspiration channel from the tip of a laryngeal mask to the outside of the laryngeal mask. Due to this gastric channel, laryngeal masks are currently classified into: first generation masks, which are those that do not have a gastric aspiration channel, and second generation masks, which are those that do have a gastric aspiration channel.

It has been observed that in certain cases the gastric channel of the second generation laryngeal masks alone is not sufficient and other methods are needed to contain the secretions at the outlet of the oesophagus, and to prevent said secretions, once inside the dome of the laryngeal mask, from returning backwards on their steps and passing to the patient's lungs, creating severe pneumonia by gastric aspiration.

It has also been observed that, over the years, laryngeal masks have been being made with an increased number of tubes that go from the outside of the laryngeal mask to the inside of the laryngeal mask. The increase in the number of said tubes of which the laryngeal masks of new generations consist is intended to bring new functionalities to them: for example, there are masks that have, in addition to one or two tubes for gases to enter and exit the lungs, other tubes or channels intended for the excretion of any gastric secretions that may reach the dome of the mask, others intended to insert different types of devices into the larynx of the patient such as cables which have photographic or video cameras at the end, medical devices with needles for biopsies, echocardiography devices, and so on. This has been causing a problem that has only grown with the number of tubes, i.e. an increase in the space occupied by said tubes within a limited space such as the diameter of the mouth or larynx: with the increase in space occupied by the tubes, not only is it difficult for the practising physician to correctly insert and handle a mask with multiple tubes into the patient's mouth and larynx, but there is also the fact that there is less free space within the larynx/inside the tubes, which can cause problems in terms of sealing the laryngeal mask off from the laryngeal and oesophagus, an overflow of secretions, the impossibility of passing any instrument outside the laryngeal mask into the oesophagus, such as gastric probes or oesophagoscopes.

### Description of the invention

In order to solve the above-mentioned disadvantages, the present invention refers to a laryngeal mask with improved characteristics compared to conventional laryngeal masks.

The laryngeal mask incorporates a dome and a system of connection channels, including a ventilation channel configured to produce artificial ventilation for a patient by inserting air into the patient's larynx through said ventilation channel and said dome.

The laryngeal mask additionally includes a gastric aspiration tube input, being said input located at a distal end of the dome, passing through the lateral wall of said dome until reaching the proximal end of the system of connection channels to the exit of the laryngeal mask, exterior to the patient

The gastric aspiration tube input is configured to absorb any gastric secretions from the patient's oesophagus.

Novelly, in the laryngeal mask according to the invention, at least two of the tubes comprising the mask, and preferably a multiplicity of them or even all of them, are partially embedded one into the other along their entire length, so that the area or space occupied by the tubes in this configuration is significantly less than the space they would occupy if the mask consisted of completely circular tubes with the same diameters but which were tubes independent of each other, contiguous. Colloquially explained, tubes "steal" a portion of area or section from one or more of their contiguous tubes, losing at least part of their regular and symmetrical circular section, and adopting a section with a geometrically irregular, asymmetrical and non-circular shape. By means of an adequate selection of the areas of the sections resulting from the area which are "thefts" of the different contiguous tubes, it is possible to obtain a configuration wherein each of the tubes has a section or area large enough to be able to fulfil its corresponding task of allowing the exit and entry of gases and the exit of secretions, but at the same time resulting in a significant reduction in the overall thickness of all the tubes within the mouth and larynx, thus significantly alleviating the above-mentioned problems relating to the high volume occupied by all the tubes of the prior art within the mouth and larynx of patients.

Therefore, according to the present invention, for the first time sets of channels or tubes of connections from the outside to the dome are created inside the laryngeal mask that are defined as asymmetric, irregular, and not circular, creating imprints of some channels or tubes inside the other contiguous area "stealing" sections.

Also novelly, the laryngeal mask incorporates at least a gastric flow non-return preventer located at the distal end of the dome. Said non-return preventer incorporates at least a flexible membrane, and preferably two or more flexible membranes, either facing each other horizontally or facing each other obliquely at an angle to allow fluid to flow in a single direction from the outside to the inside of the dome, but not in the opposite direction.

Preferably, the laryngeal mask includes at least a first ring or prominence located around the inlet of the gastric aspiration tube. Preferably, there is a plurality of first rings located around the inlet of the gastric aspiration duct. Said at least a first ring is configured to fit the walls of the oesophagus and the external oesophageal ring to prevent gastric secretions from flowing between the wall of the laryngeal mask and the wall of the oesophagus, thus forcing said gastric secretions to flow through the inlet of the gastric aspiration tube.

Also preferably, the laryngeal mask includes at least a second retention ring around the perimeter of the dome. Said at least a second ring is configured to fit the wall of the patient's larynx to prevent gastric secretions from flowing between the wall of the laryngeal mask and the wall of the oesophagus, thus forcing said gastric secretions to flow through the inlet of the gastric aspiration tube.

Preferably, the inside of the dome of the laryngeal mask includes at least an "anti-secretion" barrier or wall, which protects from the passage of secretions from the oesophagus to the laryngeal mask, and from the laryngeal mask to the lungs.

Likewise, preferably, the interior of the dome of the laryngeal mask includes at least a "pool" or "reservoir", which are hollow enclosures at least partially closed within the dome, configured to retain, either temporarily, gastric secretions from the oesophagus, making it difficult or at least delaying their return to the lungs.

Depending on a possible embodiment of the laryngeal mask, the non-return valve system comprises two membranes that have a certain angle of attack, and the membranes are therefore obliquely opposed to each other. This favours the retentive and "non-return" action of said valve. Alternatively, it is also possible for both membranes to be in the same plane and parallel to each other, facing each other.

### Brief description of the figures

As part of the explanation of at least an embodiment of the invention the following figures have been included.
Figure 1: Schematically shows how a laryngeal mask is placed in the laryngeal area of a patient.
Figure 2: Shows a perspective view of an embodiment of the laryngeal mask, including the non-return system to prevent secretions from the oesophagus from leaking into the trachea.
Figure 3: Shows a frontal view of the laryngeal mask, wherein the first rings or prominences around the inlet of the gastric aspiration tube can be observed.
Figure 4: Shows a detailed view of the first rings around the inlet of the gastric aspiration tube.
Figure 5: Shows a perspective view of the inside of the dome, with the barriers and the gastric secretion retention pool.
Figure 6: Shows a section view of an embodiment of the aerial tubes of a laryngeal mask according to the invention, showing that said tubes have an irregular and asymmetrical section.

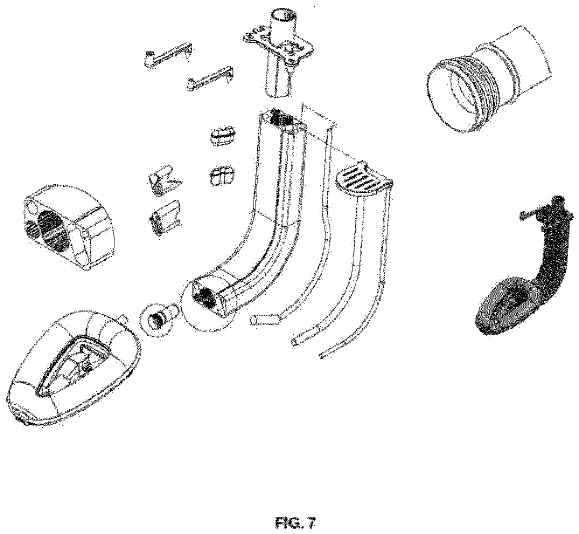

Figure 7: Shows a quartering view of a laryngeal mask.

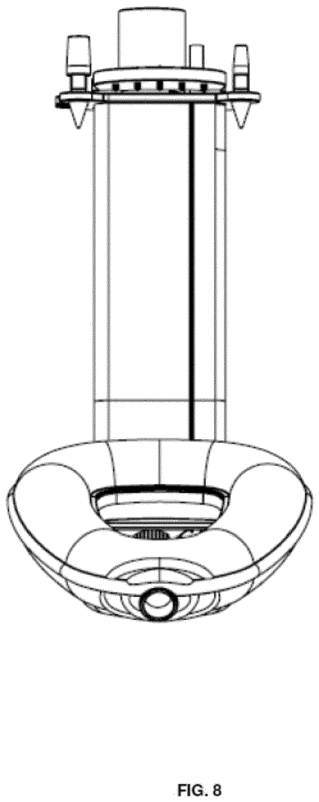

Figure 8: Shows a front view of a laryngeal mask.

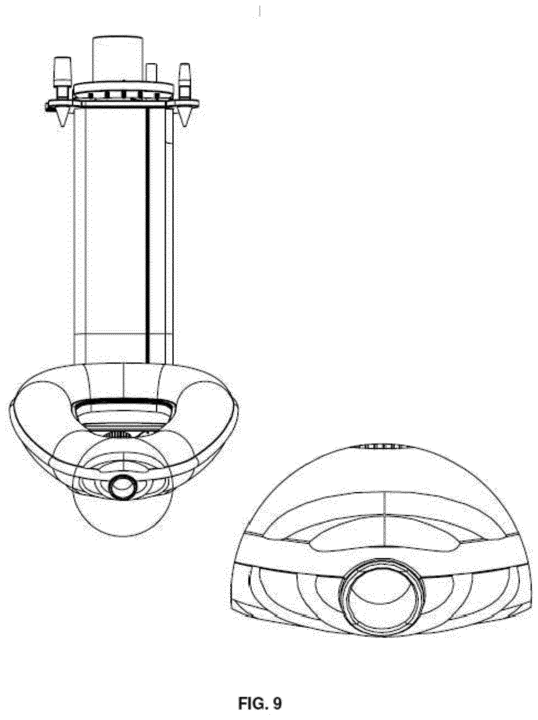

Figure 9: Shows a frontal view and a frontal detail of the dome of the laryngeal mask.

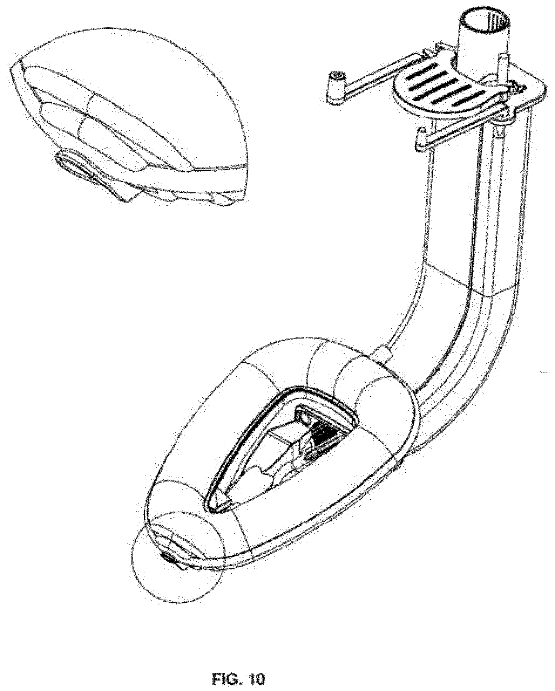

Figure 10: Shows a perspective view of a mask according to the invention, as well as a detailed view, also in perspective, of the front of the dome.

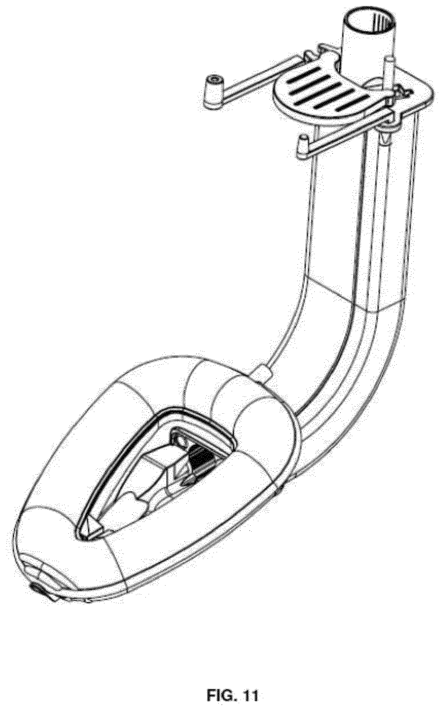

Figure 11: Shows another perspective view of a laryngeal mask embodiment.

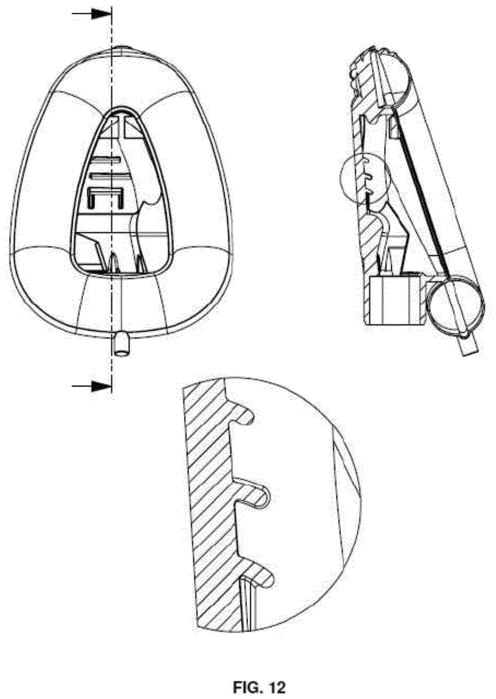

Figure 12: Shows different views of the dome.

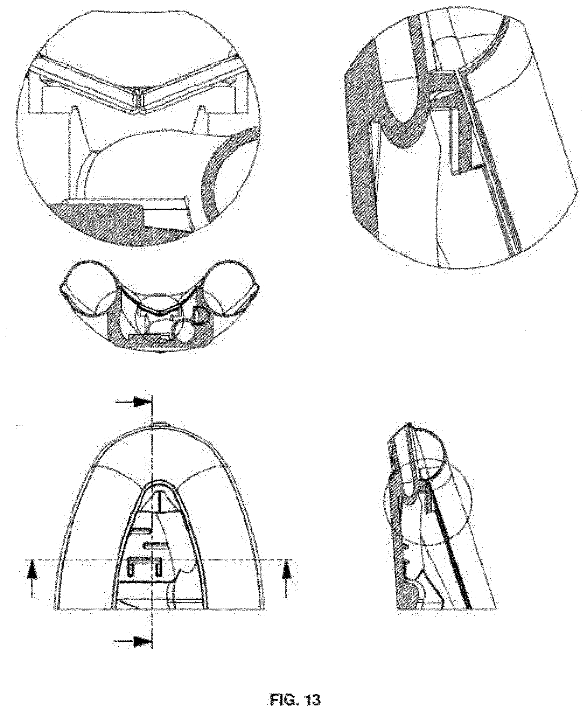

Figure 13: Shows different views and sections of the dome.

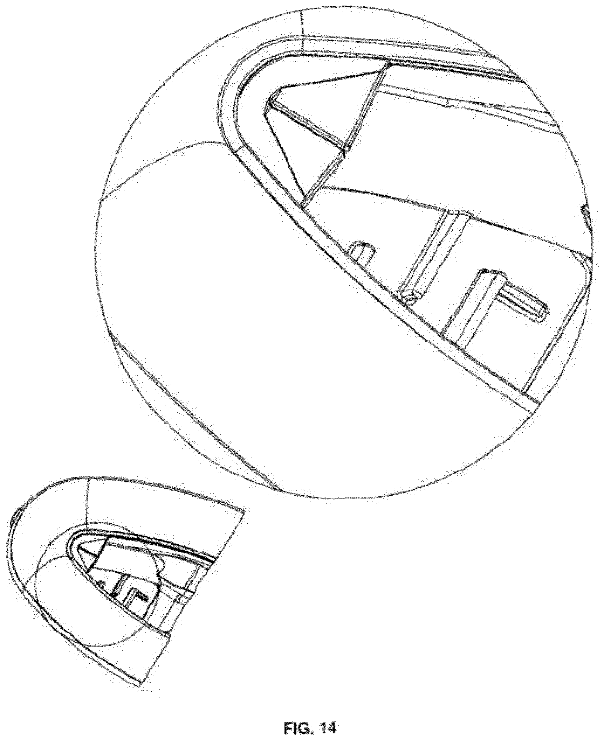

Figure 14: Shows a detail of the dome wherein the valve system and the pools can be seen.

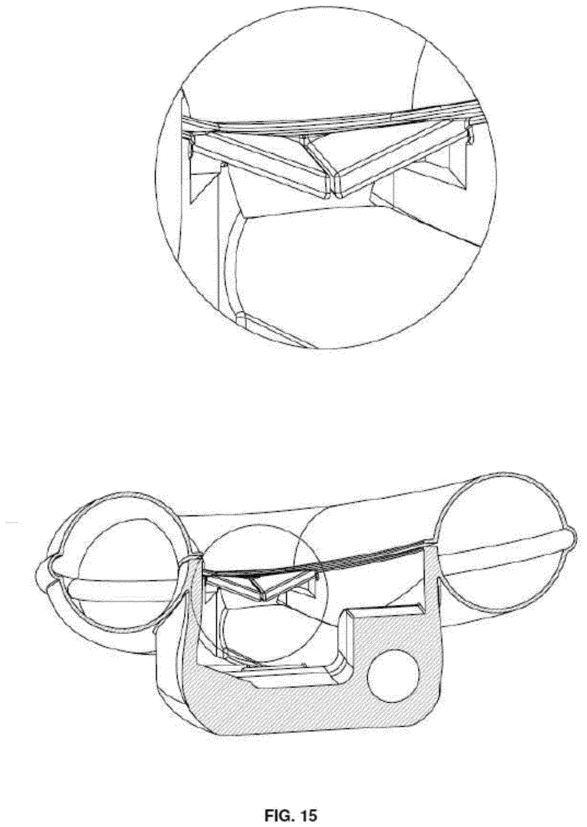

Figure 15: Shows a section of the dome wherein the valve system is observed.

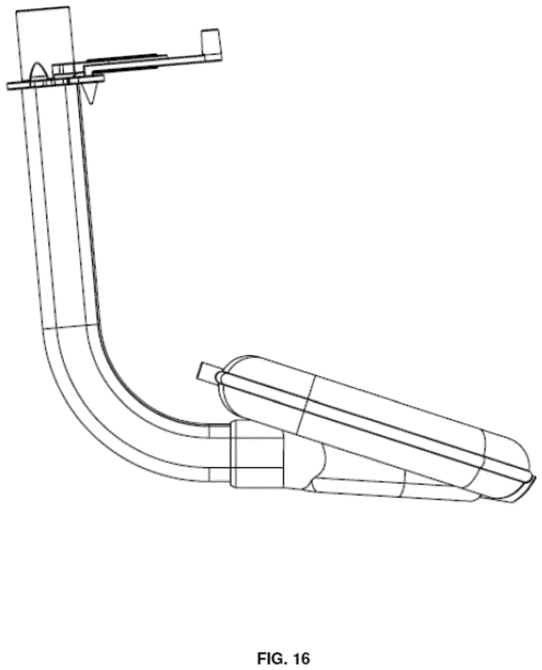

Figure 16: Shows a lateral view of the laryngeal mask.

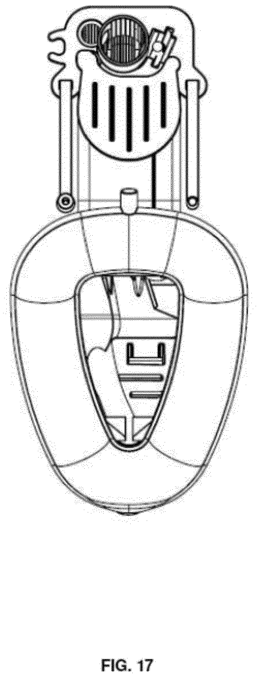

Figure 17: Displays a top view of the laryngeal mask.

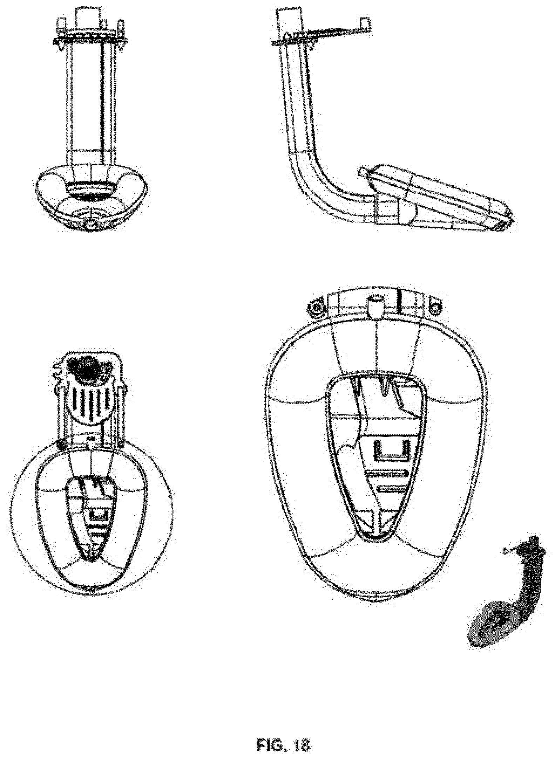

Figure 18: Shows lateral, frontal and superior views of the laryngeal mask.

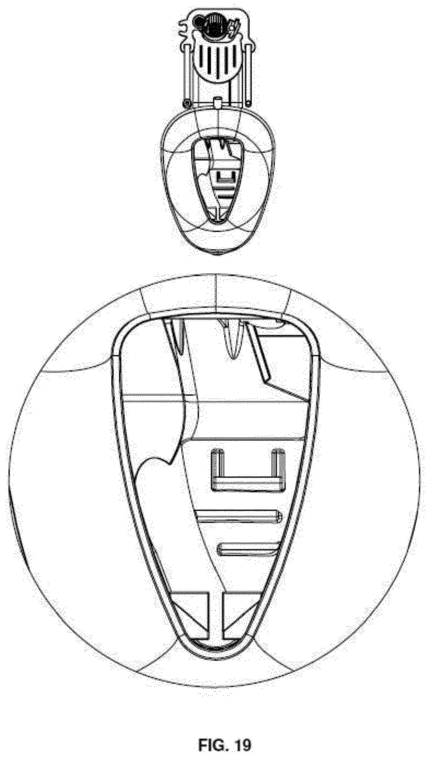

Figure 19: Shows a top view of the mask and dome.

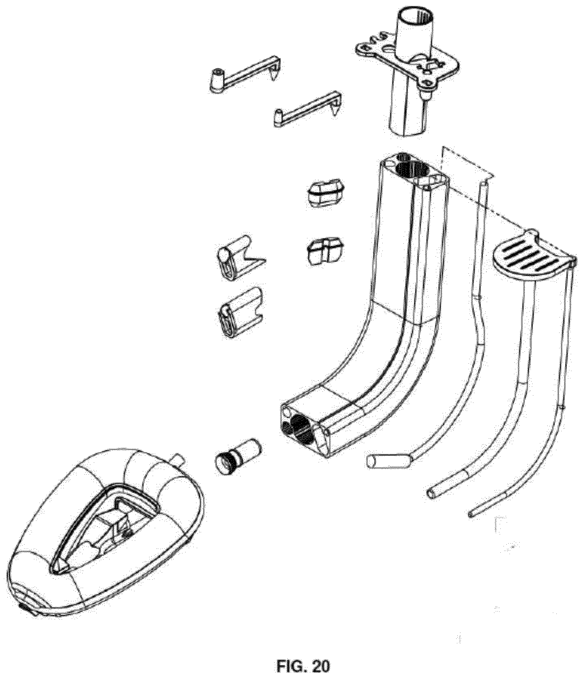

Figure 20: Shows a quartering view of the laryngeal mask.

### Detailed description

The present invention refers, as mentioned above, to a laryngeal mask (1).

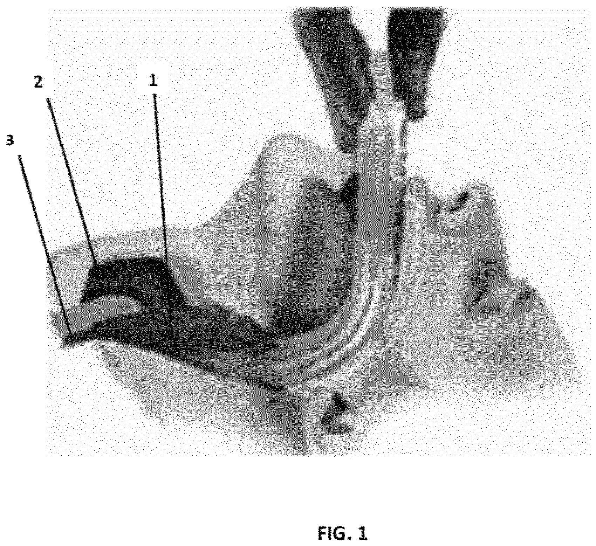

Figure 1 shows a sketch of the conventional mode of placement of a laryngeal mask (1) in a patient's larynx. The trachea (2) and oesophagus (3) are also shown. The most distal part of the laryngeal mask (1) is inserted into the patient's oesophagus (3).

The laryngeal mask (1) incorporates in the distal part of its dome an anti-return system composed, in this case, of a valve (4), preferably located in the most distal area of said laryngeal mask (1), at the end of the dome (5) intended to be located in the larynx (2), just opposite the inlet to the oesophagus.

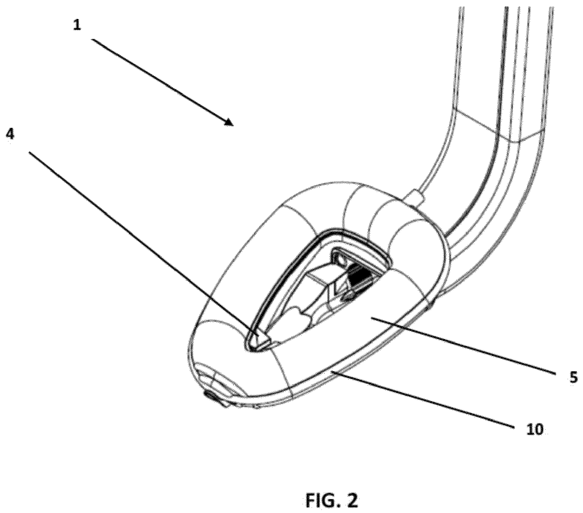

Figure 2 shows a preferred embodiment of the laryngeal mask (1) with a non-return valve (4).

According to another preferred embodiment of the laryngeal mask (1), the valve (4) has two membranes. The two diaphragms of the valve (4) act as a spring, thus achieving an optimum sealing of the dome section (5) covered by the valve (4).

The valve (4) can cover a larger or smaller section of the dome (5), depending on the needs and/or characteristics of the type of patient (depending on age, sex, etc.).

At rest, the membranes of the valve (4) have their closest edges in contact with each other or are very close to each other.

The membranes allow the fluid to pass in one direction only, preventing the flow in the opposite direction when they are in the resting position.

The membranes have high elasticity and flexibility properties. Each membrane is a rotation/torsion element, so that when the fluid (gastric secretions) circulates in a certain direction, the membranes expand and allow the fluid to pass, but by preserving the flexibility of its material the mechanical energy at the time of the rotation, the membranes return to their resting position and close when the fluid does not circulate in that direction avoiding the return fluid.

Depending on a particular embodiment, the membranes may have a certain angle of attack, rather than being in the same plane. By means of this angle of attack between them, the one-way flow effect of the valve (4) diaphragms can also be achieved.

Alternatively, the membranes can also be parallel to each other in the same plane.

Depending on a possible embodiment, the laryngeal mask (1) incorporates inside and/or at the bottom of the dome (5) at least an anti-secretion barrier OR WALL (6), which protects, either partially and/or temporarily, from the passage of secretions from the oesophagus (3) to the laryngeal mask (1) and from the laryngeal mask (1) to the lungs. This/these barrier(s) (6) favour(s) the passage of fluid in a single direction, which is the opposite direction to the input of the lungs.

Also, according to a possible embodiment, the laryngeal mask (1) includes a pool (7) or hollow and empty reservoir inside and/or at the bottom of the dome (5). This pool (7) is used to collect and retain any secretions from the oesophagus (3).

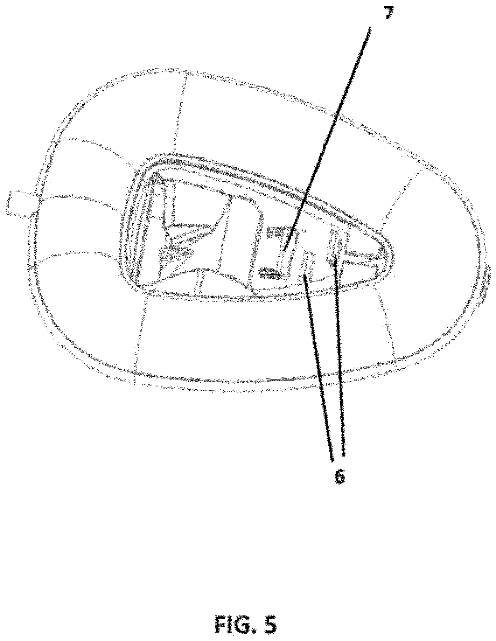

Figure 5 shows said barriers (6) and said pool (7).

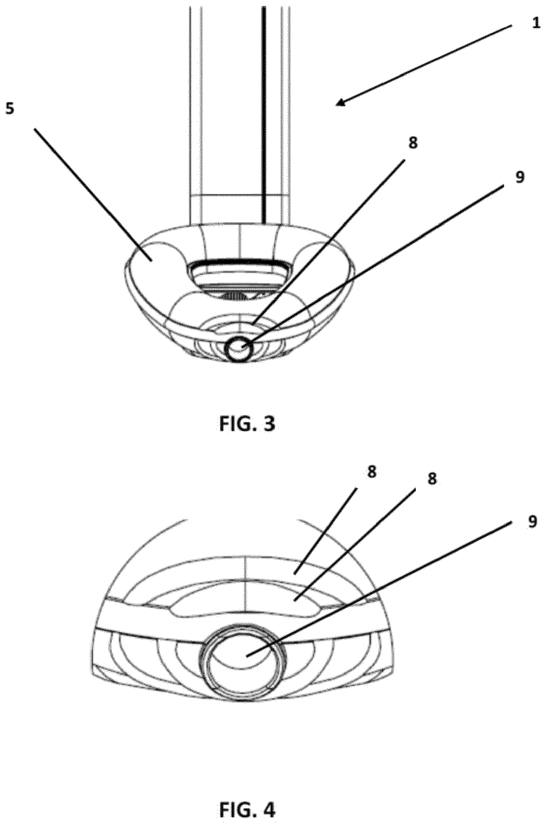

Also depending on a possible embodiment (see Figure 3 and Figure 4), the laryngeal mask (1) comprises at least a first ring (8) or prominence around the inlet (9) of the gastric aspiration tube at the tip of the laryngeal mask (1).

When the laryngeal mask (1) is already in its correct position in the patient's larynx (2), with the tip of the laryngeal mask (1) inside the oesophagus (3) (specifically inside the upper oesophageal ring), the said ring(s) is/are fitted to the walls of the oesophagus (3) to prevent secretions from leaving the lateral wall between the laryngeal mask (1) and the wall of the oesophagus (3), thus forcing any said secretions out through the inlet (9) of the gastric aspiration tube.

Also, depending on a possible embodiment, the laryngeal mask (1) includes at least a second ring (10) that perimetrally surrounds the entire dome (5), in such a way that when the laryngeal mask (1) adjusts to the larynx (2) of the patient, said second ring (10) hinders the passage of gastric secretions between the wall of the oesophagus (3), the larynx and the wall of the laryngeal mask (1).

The laryngeal mask (1) incorporates a plurality of access channels from the outside of the laryngeal mask (1) that connect with the dome (5) of the laryngeal mask (1) with many different functionalities in order to be able to aspirate or to be able to insert through them an aspiration probe, to clean the dome (5) from the dangerous gastric secretions, to insert fibroscopes, diagnostic and biopsy means such as puncture needles, temperature probes or pressure probes.

Inside at least a of said channels, the laryngeal mask (1) can incorporate a continuous vision system to be able to visualise from the outside whether or not there are gastric secretions accumulated inside the dome (5).

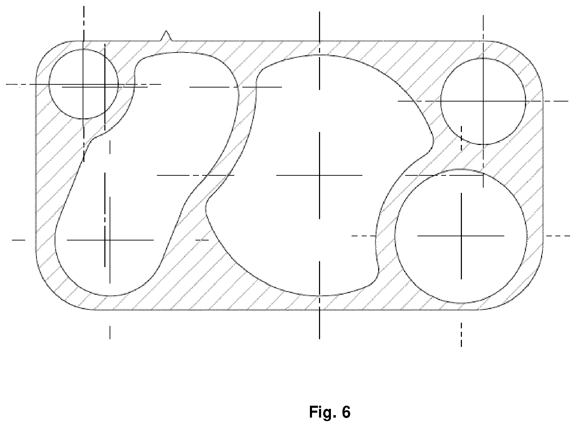

Figure 6 shows the section of the tube assembly consisting of the mask according to a preferred embodiment of the invention. According to this preferred embodiment, the mask consists of five tubes in total, among which are the tubes for the inlet and outlet of gases of the patient, among others.

In the configuration shown in this figure, the area of each of the channels and the thickness of the tubes as a whole has been optimised so that two of them still retain a circular section, while the other three have an asymmetric, non-circular and irregular geometric section, which allows them to occupy a smaller amount of space or total volume than if they had a strictly symmetrical circular section and were contiguous to each other. This configuration allows an optimisation of the total space or total volume occupied by the set of pipes or connection channels without this being detrimental to the section or area of the pipes, and therefore to the flow of gases or flows or liquids or secretions that they may carry. Channels or connection tubes with asymmetric, irregular, and non-circular configurations occupy less volume or space in the patient's mouth or larynx than with the same number of channels or tubes if they are contiguous to each other and are circular, symmetrical, and regular. Allowing them to enter smaller mouths and larynges anatomically.

In general, the mask in accordance with the invention comprises a plurality of tubes, of which at least one of them has a section of irregular geometrical shape, although the number of tubes with a section of asymmetrical and non-circular irregular geometrical shape may be any number from one up to the totality of the tubes of which the mask consists.

The axes of the tubes of which the mask consists are parallel and exhibit an axial eccentricity.

Also, in a general way, the reduction of sections or areas in those pipes which have irregular, asymmetric and non-circular geometric shapes with respect to the section of a pipe of the same maximum diameter and which has a section of circular geometric shape between 10% and 40%, preferably between 20% and 30%, and most preferably around 25% of reduced area.

By means of an adequate selection of the areas of the "stolen" sections or imprints or foods of the different adjacent tubes it is possible to obtain a configuration wherein each one of the tubes has a section or area sufficiently large to be able to fulfil its corresponding objective for the passage of gases towards the inside and outside and of output of liquids or secretions, but resulting at the same time in a significant reduction in the total space or total volume or thickness occupied jointly by all the tubes within the larynx, thus significantly alleviating the above-mentioned problems relative to the high volume or space occupied by the tubes of the prior art wherein the tubes to adjacent channels are circular and symmetrical occupying a large volume and space within the limited space of the patients' mouth and larynx.

## Claims

1. Laryngeal mask (1) comprising a dome and a plurality of tubes for the airways connected to said mask, **characterised in that** the axes of the tubes are parallel, have an axial eccentricity, and at least one of them has a section with an irregular, asymmetric and non-circular geometric shape.

2. Laryngeal mask (1) according to claim 1, wherein one of the tubes is a ventilation tube for the entry of air or other gases into the patient's lungs.

3. Laryngeal mask (1) according to claims 1 or 2, wherein one of the tubes is an intubation tube for the exit of gases from the lungs to the outside.

4. Laryngeal mask (1) according to any of the claims 1 to 3, wherein one of the tubes is a gastroesophageal tube, for the outflow of fluids from the oesophagus to the exterior of the patient.

5. Laryngeal mask (1) according to any of the preceding claims, wherein the section reduction of at least a tube having an irregular geometric shape with respect to the section of a tube of the same maximum diameter and having a section of circular shape is between 10% and 40%.

6. Laryngeal mask (1) according to claim 5, wherein the section reduction of at least a pipe having an irregular geometric shape with respect to the section of a pipe of the same maximum diameter and having a circular shape section is between 20% and 30%.

7. Laryngeal mask (1) according to claim 5, wherein the section reduction of at least a pipe having an irregular geometric shape with respect to the section of a pipe of the same maximum diameter and having a circular shape section is 25%.

8. Laryngeal mask (1) comprising a dome (5) and a ventilation channel configured to produce the artificial ventilation of a patient by introducing air into the patient's larynx (2) through said ventilation channel and said dome (5), wherein the laryngeal mask (1) is **characterised by** comprising at least a non-return system (4) at the distal end of the dome (5), wherein the system (4) comprises at least two flexible membranes configured to allow fluid to flow in a single direction from the outside to the inside of the dome (5).

9. Laryngeal mask according to claim 8, further comprising an inlet (9) of a gastric aspiration tube, said inlet (9) being located at a distal end of the dome (5), in the lateral wall of said dome (5), wherein said inlet (9) is configured to absorb any gastric secretions from the patient's oesophagus (3).

10. Laryngeal mask (1) according to claims 8 or 9, comprising at least one first ring (8) around the inlet (9) of the gastric aspiration tube, wherein said at least one first ring (8) is configured to fit the walls of the oesophagus (3) to prevent gastric secretions from flowing between the wall of the laryngeal mask (1) and the wall of the oesophagus (3), thus forcing said gastric secretions to flow through the inlet (9) of the gastric aspiration tube.

11. Laryngeal mask (1) according to any of the preceding claims 8 to 10, comprising at least one second ring (10) around the perimeter of the dome (5), and which is configured to fit the patient's larynx (2) to prevent gastric secretions from flowing between the wall of the laryngeal mask (1) and the wall of the oesophagus (3), thus forcing said gastric secretions to flow through the inlet (9) of the gastric aspiration tube.

12. Laryngeal mask (1) according to any of the preceding claims 8 to 11, comprising inside the dome (5) at least an anti-secretion barrier (6), which protects from the passage of secretions from the oesophagus (3) to the laryngeal mask (1), and from the laryngeal mask (1) to the lungs.

13. Laryngeal mask (1) according to any of the preceding claims 8 to 12, comprising a pool (7) at least partially closed inside the dome (5), wherein said pool (7) is configured to retain at least temporarily gastric secretions from the oesophagus (3).

14. Laryngeal mask (1) according to any of the preceding claims 8 to 13, wherein the non-return system (4) comprises two membranes which have an oblique angle to each other.

15. Laryngeal mask (1) according to any of the preceding claims 8 to 13, wherein the non-return system (4) comprises two membranes parallel to each other, facing each other's edges horizontally.
